# EUROPEAN PATENT APPLICATION

(11) **EP 3 881 795 A1**
(43) Date of publication of application: **22.09.2021**
(21) Application number: 20305292.3
(22) Date of filing: 20.03.2020
(51) Int. Cl.: A61C 7/00, A61C 7/08, G16H 50/50, A61C 9/00

(54) **METHODS FOR MANAGING RELAPSE IN ORTHODONTICS**

(71) Applicant: Carestream Dental LLC, Atlanta, GA 30339 (US)
(72) Inventor: REYNARD, Delphine, Marne La Vallee Cedex 2 Croissy-Beaubourg 77435 (FR)
(74) Representative: Wagner & Geyer

(57) **Abstract**

The present disclosure describes methods for determining an orthodontic virtual setup for a patient who has experienced relapse after previous orthodontic treatment. According to example embodiments, the methods use and begin with a 3D model in which the patient's teeth are in an arrangement corresponding to the end of the immediately previous orthodontic treatment. In this 3D model, the positions of the patient's teeth are known to be correct and it is also known that it is possible for the patient's teeth to be moved into such positions. If the prior 3D model has been segmented, the practitioner does not need to manually move the teeth into desired positions to create a virtual setup because the patient's teeth are already in their desired positions and arrangement.

## Description

### FIELD OF THE INVENTION

The present invention generally relates the field of dental orthodontics and, more particularly, to the orthodontic treatment of relapse using three-dimensional imaging and modeling techniques.

### BACKGROUND

In today's orthodontics, before orthodontic treatment for a patient commences, a virtual three-dimensional ("3D") model of the initial tooth arrangement of the patient's upper and lower jaws is often created. Many techniques may be used to acquire or generate a 3D model of the patient's teeth. In one approach, the patient's mouth can be scanned using an intraoral camera that uses triangulation, for example, acquiring multiple image frames before reconstructing a 3D surface model of the teeth. Alternatively, positive physical casts representing the patient's upper and lower jaws can also be scanned by the intraoral camera to generate the 3D surface model of the teeth. Additionally, the positive physical casts may be scanned using a cone beam computed tomography ("CBCT") x-ray device to generate a 3D volume model of the teeth.

After creation, the 3D model is segmented into individual virtual teeth and the teeth may then be displaced relative to each other by a practitioner (e.g., a dentist or orthodontist) to form a desired target or final arrangement of the patient's teeth ("virtual setup") after orthodontic treatment. Numerous applications have been developed and are now available to generate a virtual setup. One of the aims of the virtual setup is to determine whether or not there is sufficient space in the patient's maxillary and mandibular jaws for all the teeth. Another aim of the virtual setup is to model an aid in visualizing treatment parameters like tooth size-arch length discrepancy and to determine whether or not intra-proximal reduction, extraction, or surgery are required. Once the virtual setup has been created, the practitioner then uses the virtual setup to aid in planning an orthodontic treatment sequence for the patient. If the practitioner decides that aligner treatment is appropriate for the patient, the practitioner can use the virtual setup to decompose tooth movements and generate aligner appliances for the various steps of the aligner treatment. Alternatively, if the practitioner decides that conventional bracket and wire braces are appropriate for the patient, the practitioner can use the virtual setup for indirect bonding of the brackets to the patient's teeth.

When generating a virtual setup there is always a risk that the patient's teeth may not reach their desired positions and arrangement after treatment. This failure to reach their desired positions may be the result of a variety of causes, including, but not limited to collisions between tooth roots. For patients who have completed orthodontic treatment, teeth have a tendency to move back toward the original malocclusion as a result of periodontal, gingival, occlusal and growth-related factors. Hence, a problem may arise with a patient maintaining his/her lower front teeth in their corrected positions and arrangement. This problem, often referred to as "relapse", may occur after orthodontic treatment if no retainer is worn by the patient, if the patient fails to wear his/her retainer as instructed, or if a retainer wire has broken. When relapse occurs and the patient's teeth must receive further orthodontic treatment in order to be moved into their desired positions or arrangement, the practitioner must create a new orthodontic treatment plan based on a new virtual setup. Unfortunately, the creation of a new virtual setup for treating relapse requires the expenditure of time and money. And, such new virtual setup needs to be easily reachable.

There is, therefore, a need in the industry for a method of determining a virtual setup for patients experiencing relapse after orthodontic treatment that minimizes the amount of time and money needed to create a new virtual setup that is easily reachable, and that solves this and other problems, difficulties, deficiencies, and shortcomings of present methods.

### SUMMARY

Broadly described, the present invention comprises methods for determining an orthodontic virtual setup for a patient who has experienced relapse after orthodontic treatment. According to example embodiments, the methods use and begin with a 3D model in which the patient's teeth are in an arrangement corresponding to the end of the immediately prior orthodontic treatment. In this 3D model, the positions and orientation of the patient's teeth are known to be correct and it is also known that it is possible for the patient's teeth to be moved into such positions and orientations. If the prior 3D model has been segmented, the practitioner does not need to manually move the teeth into desired positions to create a virtual setup because the patient's teeth are already in their desired arrangement.

Advantageously, the orthodontic virtual setup created by the methods of the present invention may be used in connection with subsequent orthodontic treatments, including, but not limited to, those orthodontic treatments using orthodontic aligners or conventional orthodontic braces having brackets and wires. Further, such orthodontic virtual setup may be used as part of a method of indirect bonding the brackets to the patient's teeth.

Other uses, advantages and benefits of the present invention may become apparent upon reading and understanding the present specification when taken in conjunction with the appended drawings.

### BRIEF DESCRIPTION OF THE DRAWINGS

Fig. 1 displays pictorial representations of a three-dimensional virtual model of a patient's teeth at a current time, a three-dimensional virtual model of the patient's teeth at a previous time corresponding to the end of a previous orthodontic treatment, and the three-dimensional virtual model of the patient's teeth at a previous time registered relative to the three-dimensional virtual model of the patient's teeth at the current time.
Fig. 2 displays a flowchart representation of a method for determining an orthodontic virtual setup and intermediate orthodontic treatment steps according to an example embodiment of the present invention.
Fig. 3 displays a flowchart representation of a method for determining an orthodontic virtual setup and intermediate orthodontic treatment steps according to a first alternative example embodiment of the present invention.

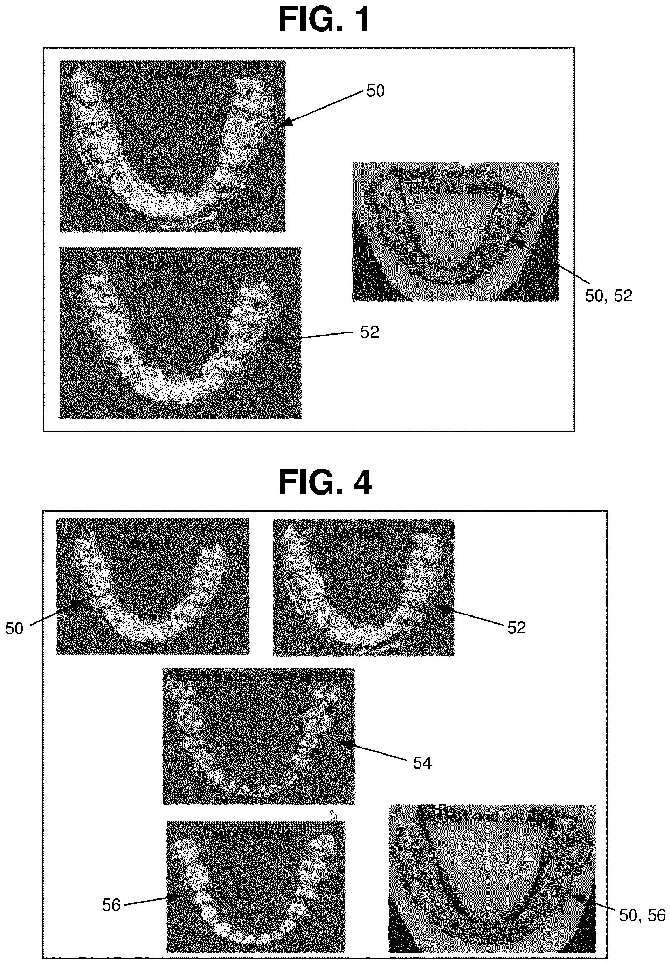

Fig. 4 displays pictorial representations of a three-dimensional virtual model of a patient's teeth at a current time, a three-dimensional virtual model of the patient's teeth at a previous time corresponding to the end of a previous orthodontic treatment, the three-dimensional virtual model of the patient's teeth at the previous time registered relative to the three-dimensional virtual model of the patient's teeth at the current time, an orthodontic virtual setup of the patient's teeth for subsequent orthodontic treatment, and the three-dimensional virtual model of the patient's teeth at the current time overlaid upon the orthodontic virtual setup.
Fig. 5 displays a flowchart representation of a method for determining an orthodontic virtual setup and intermediate orthodontic treatment steps according to a second alternative example embodiment of the present invention.
Fig. 6 displays a flowchart representation of a method of tooth-by-tooth registration for registering each tooth of a three-dimensional virtual model of the patient's teeth at a current time with the corresponding tooth of a three-dimensional virtual model of the patient's teeth at the end of a previous orthodontic treatment in accordance with an example embodiment of the present invention.
Fig. 7 displays a flowchart representation of a method for finalizing the orthodontic virtual setup to be used for subsequent orthodontic treatment.

### DETAILED DESCRIPTION OF EXAMPLE EMBODIMENTS

In the following detailed description of example embodiments of the present invention reference is made to the drawings in which like reference numerals identify like elements or steps throughout the several views. As used herein and in the drawings, the term "3D Mesh 1" (sometimes also referred to herein as "Model 1" or "initial model 50") identifies and refers to a 3D virtual model of a patient's teeth acquired and created at the current time ("time 1") (see Fig. 1) and the term "3D Mesh 2" (sometimes also referred to herein as "Model 2" or "previous model 52") identifies and refers to a 3D virtual model of a patient's teeth taken at a previous time ("time 2") corresponding to the end of a previous orthodontic treatment when the patient's teeth were positioned and arranged in their desired positions and arrangement (see Fig. 1). Relapse has occurred during the time between the acquisition and creation of the 3D Mesh 2 model and the acquisition and creation of the 3D Mesh 1 model. Also, as used herein, the term "arrangement" identifies and refers to the position and orientation of the patient's teeth.

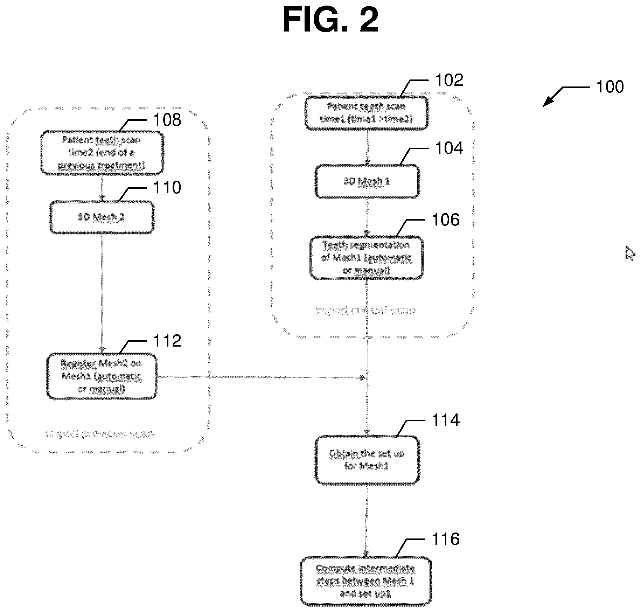

Fig. 2 displays a flowchart representation of a method 100, according to an example embodiment of the present invention, for determining an orthodontic virtual setup and intermediate orthodontic treatment steps for a patient who has experienced relapse after a previous orthodontic treatment. As illustrated in Fig. 2, the method 100 comprises acquiring a new 3D scan and corresponding representative data of the patient's teeth at the current time ("time 1") at step 102. The 3D scan may be acquired via an intraoral scan of the patient's teeth by an intraoral scanner, a cone beam computed tomography ("CBCT") scan of the patient's teeth by a CBCT device, a CBCT scan of an impression or plaster cast of the patient's teeth by a CBCT device, and/or other methods, devices, or combinations thereof available now or in the future. Continuing at step 104, initial model 50 is created from the data obtained during the 3D scan(s) (see Fig. 1). Once initial model 50 has been created, the data of initial model 50 is segmented, at step 106, into data corresponding to and representative of individual teeth. According to various example embodiments, teeth segmentation may be performed automatically, manually, or semi-automatically.

At step 108, data obtained from a 3D scan of the patient's teeth made at a previous time ("time 2") (which is before the current time ("time 1")) is retrieved from a computer readable storage medium. Similar to the 3D scan at step 102 described above, the 3D scan may have been acquired via an intraoral scan of the patient's teeth by an intraoral scanner, a cone beam computed tomography ("CBCT") scan of the patient's teeth by a CBCT device, a CBCT scan of an impression or plaster cast of the patient's teeth by a CBCT device, and/or other methods, devices, or combinations thereof then available. In the 3D scan at time 2, the patient's teeth were in their desired positions and/or arrangement at the end of the previous orthodontic treatment. Alternatively, data may be obtained from a new 3D scan of an impression or plaster cast of the patient's teeth that was made at the end of the previous orthodontic treatment.

Next, at step 110, previous model 52 is created from the data obtained during the 3D scan(s) made at step 108. It should be appreciated and understood that if the previous model 52 has been stored previously on a storage device, steps 108 and 110 of method 100 may be replaced by a single step of retrieving previous model 52 from a computer readable storage medium. Regardless of how previous model 52 is obtained, it should be appreciated and understood that previous model 52 represents the patient's teeth in their desired positions and arrangement. After the previous model 52 has been created or retrieved, previous model 52 is registered on and relative to the initial model 50 at step 112.

Advancing to step 114 of method 100, the orthodontic virtual setup for subsequent orthodontic treatment of the patient's teeth is obtained. It should be understood that in the orthodontic virtual setup, the positions and/or arrangement of the patient's teeth generally correspond to their positions and/or arrangement at the end of the previous orthodontic treatment at time 2. Such positions and/or arrangement correspond to the desired positions and/or arrangement for the patient's teeth and are known to be correct and achievable. The orthodontic virtual setup may be obtained by retrieving it from a computer readable storage medium on which it was stored at the end of the patient's previous orthodontic treatment at time 2 or by generating the orthodontic virtual setup from data stored previously on a computer readable storage medium at time 2 and corresponding to previous model 52 and/or corresponding 3D scan. Then, at step 116, intermediate steps for the subsequent orthodontic treatment (including, but not limited to, intermediate steps for orthodontic aligners) are determined based on the initial model 50 and the orthodontic virtual setup.

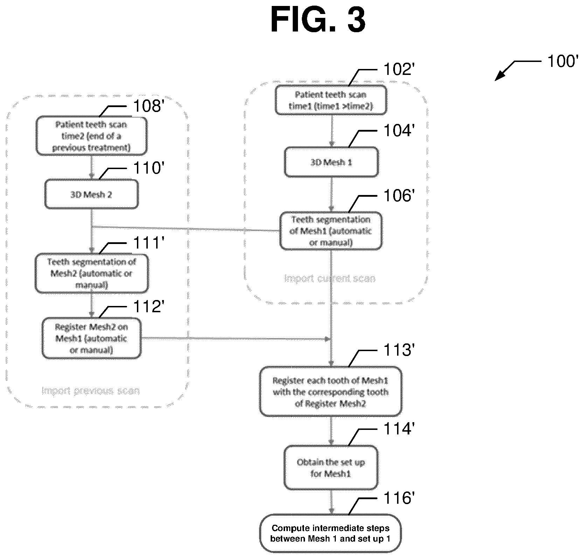

Fig. 3 displays a flowchart representation of a method 100', according to an alternative example embodiment of the present invention, for determining an orthodontic virtual setup and intermediate orthodontic treatment steps for a patient who has experienced relapse after a previous orthodontic treatment. Method 100' is substantially similar to method 100 described above with certain differences. Therefore, it is unnecessary to describe in detail here the steps that are substantially similar.

Regarding the steps of method 100' that are different than those of method 100, method 100' includes a step 111' between steps 110' and 112'. At step 111', the data of previous model 52 is segmented into data corresponding to and representative of individual teeth in their end positions and arrangement at the end of the previous orthodontic treatment. According to various example embodiments, such teeth segmentation may be performed automatically, semi-automatically, or manually. At step 113' between steps 112' and 114', each tooth of initial model 50 is registered with the corresponding tooth from previous model 52 prior to the orthodontic virtual setup for subsequent orthodontic treatment of the patient's teeth being obtained at step 114' (see registered teeth 54 of Fig. 4). In order to perform such registration, feature matching and intercuspal position ("ICP") are used in an iterative method to remove the part of the teeth that could have changed because of the scan at time 1 or because the teeth are not in the same position as at time 2 (so some part of a tooth could be hidden in one model and not in the other one). It should be appreciated and understood that other methods of registration may be used in other example embodiments.

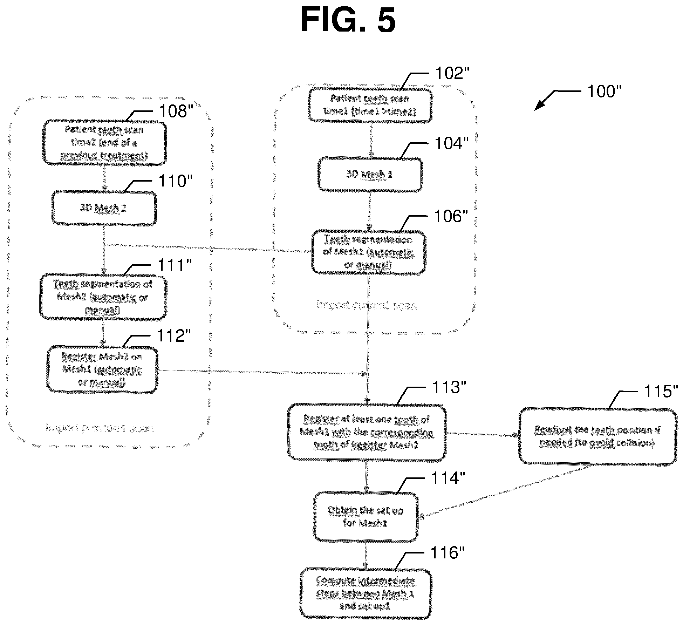

Fig. 5 displays a flowchart representation of a method 100", according to still another alternative example embodiment of the present invention, for determining an orthodontic virtual setup and intermediate orthodontic treatment steps for a patient who has experienced relapse after a previous orthodontic treatment. Method 100" is substantially similar to method 100' described above with certain differences. Therefore, it is unnecessary to describe in detail here the steps that are substantially similar.

Regarding the steps of method 100" that are different than those of method 100' described above, at step 113", at least one tooth of initial model 50 is registered with the corresponding tooth from previous model 52, thereby globally registering the initial 50 and previous model 52. Thus, in method 100", one or more teeth of initial model 50 may be registered as opposed to each of the teeth of initial model 50 being registered as in method 100'. As in method 100', feature matching and ICP may be used to perform such registration, but it may be possible for other methods to be used in other example embodiments. In addition to the difference in step 113", method 100" further includes step 115" that may optionally be performed between steps 113" and 114". At step 115", a determination is made to see (i) if teeth have actually moved between time 2 and time 1 (and, hence, between previous model 52 and initial model 50) and (ii) if there is a collision between any adjacent teeth. If either (i) or (ii) has occurred, the position and arrangement of one or more teeth in initial model 50 may be adjusted.

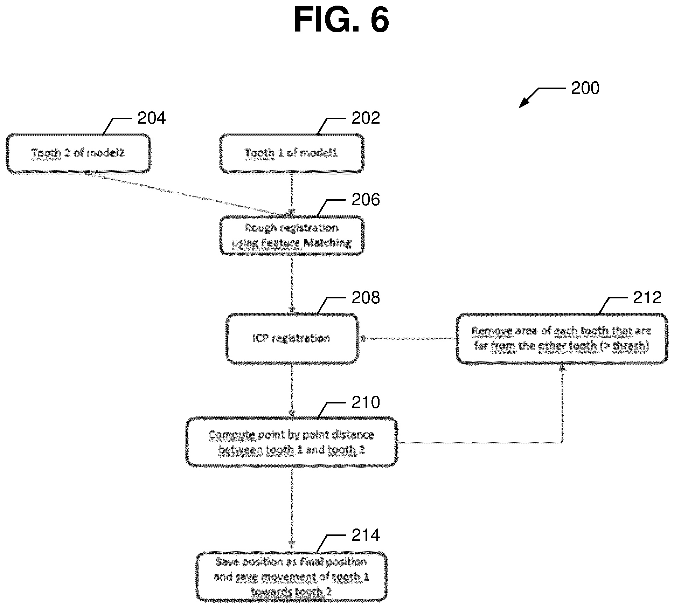

Fig. 6 displays a flowchart representation of a method 200 of tooth-by-tooth registration that is used in step 113' described above to register each tooth of initial model 50 with the corresponding tooth from previous model 52. Starting with a tooth of initial model 50 at step 202 and a corresponding tooth from previous model 52 at step 204, rough registering of the teeth is performed at step 206 by matching various features of the teeth. Next, at step 208, intercuspal position registering is performed. Then, at step 210, point-by-point distances are computed between various points on the teeth. If some of the distances are greater than a pre-determined, pre-decided threshold value, the areas of each tooth that are too far from the other tooth (i.e., those areas having distances therebetween that are greater than the pre-determined, pre-decided threshold value) are removed at step 212. Once the areas are removed, the method 200 loops back to step 208 where intercuspal position registering is again performed. If all of the distances computed at step 210 are less than the pre-determined, pre-decided threshold value, the position of the tooth from the initial model 50 is saved as its "final" position in the orthodontic virtual setup 56 (see Fig. 4) and the amount of movement of the tooth from initial model 50 toward the tooth from previous model 52 is saved.

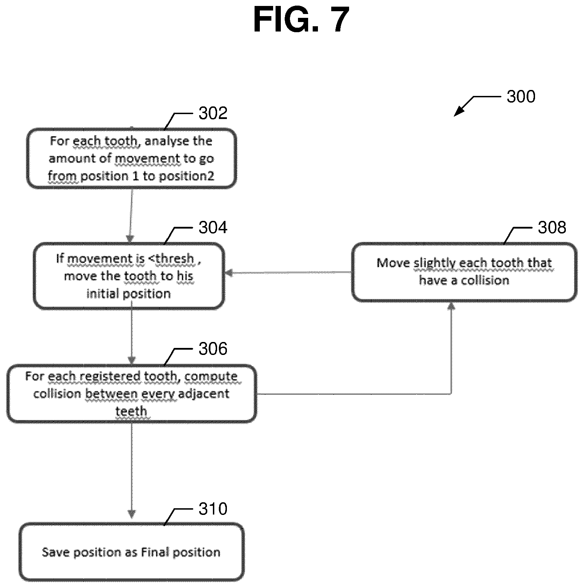

Fig. 7 displays a flowchart representation of a method 300 for finalizing the orthodontic virtual setup 56 to be used for subsequent orthodontic treatment. Method 300 is used at step 114 of method 100 as briefly described above. With the teeth of initial model 50 registered onto the teeth of previous model 52 as seen in Fig. 4, all of the teeth are analyzed at step 302 of method 300 to determine the amount of movement necessary for each tooth to move from its position in initial model 50 to its position in previous model 52. Then, at step 304, the movements necessary for each tooth are considered and if the amount of movement for a tooth is less than a pre-determined, pre-decided threshold value, the tooth is moved to its initial position. Continuing at step 306, the method 300 determines whether a collision exists for each registered tooth 54 with each of its adjacent teeth. If at least one collision exists, each tooth involved in the collision is moved slightly at step 308 and the method 300 loops back to repeat step 304. If no collision exists, the position of the teeth is saved as the final position in the orthodontic virtual setup 56. The orthodontic virtual setup 56 is shown individually and as overlaid on initial model 50 in Fig. 4.

Consistent with at least one example embodiment, the example methods may be implemented via a computer program with stored instructions that perform on image data that is accessed from an electronic memory. As can be appreciated by those skilled in the image processing arts, a computer program of an example embodiment herein may be utilized by a suitable, general-purpose computer system, such as a personal computer or workstation. However, many other types of computer systems may be used to execute the computer program of described example embodiments, including an arrangement of one or networked processors, for example.

A computer program for performing methods of certain example embodiments described herein may be stored in a computer readable storage medium. This medium may comprise, for example; magnetic storage media such as a magnetic disk such as a hard drive or removable device or magnetic tape; optical storage media such as an optical disc, optical tape, or machine-readable optical encoding; solid state electronic storage devices such as random access memory (RAM), or read only memory (ROM); or any other physical device or medium employed to store a computer program. Computer programs for performing methods of described example embodiments may also be stored on computer readable storage medium that is connected to the image processor by way of the Internet or other network or communication medium. Those skilled in the art will further readily recognize that the equivalent of such a computer program product may also be constructed in hardware.

It should be noted that the term "memory", equivalent to "computer-accessible memory" in the context of the application, may refer to any type of temporary or more enduring data storage workspace used for storing and operating upon image data and accessible to a computer system including, for example, a database. The memory could be non-volatile, using, for example, a long-term storage medium such as magnetic or optical storage. Alternately, the memory could be of a more volatile nature, using an electronic circuit, such as random-access memory (RAM) that is used as a temporary buffer or workspace by a microprocessor or other control logic processor device. Display data, for example, is typically stored in a temporary storage buffer that can be directly associated with a display device and is periodically refreshed as needed in order to provide displayed data. This temporary storage buffer can also be considered to be a memory, as the term is used in the application. Memory is also used as the data workspace for executing and storing intermediate and final results of calculations and other processing. Computer-accessible memory can be volatile, non-volatile, or a hybrid combination of volatile and non-volatile types.

It should be understood and appreciated that computer program products for the example embodiments herein may make use of various image manipulation algorithms and/or processes that are well known. It should be further understood and appreciated that example computer program product embodiments herein may embody algorithms and/or processes not specifically shown or described herein that are useful for implementation. Such algorithms and processes may include conventional utilities that are within the ordinary skill of the image processing arts. Additional aspects of such algorithms and systems, and hardware and/or software for producing and otherwise processing the images or co-operating with the computer program product of the application, are not specifically shown or described herein and may be selected from such algorithms, systems, hardware, components and elements known in the art.

It should be still further understood and appreciated that example embodiments according to the present invention may include various features described herein individually or in combination.

The invention has been described in detail herein with reference to one or more example embodiments, but it should be appreciated and understood that variations and modifications may be affected within the spirit and scope of the invention. The one or more presently disclosed example embodiments are, therefore, considered in all respects to be illustrative and not restrictive. The scope of the invention is indicated by the appended claims, and all changes that come within the meaning and range of equivalents thereof are intended to be embraced therein.

## Claims

1. A method for determining an orthodontic virtual setup for a patient experiencing relapse after a previous orthodontic treatment, the method comprising the steps of:
obtaining a first three-dimensional virtual model of a patient's teeth, wherein the patient's teeth are in an arrangement at a current time;
obtaining a second three-dimensional virtual model of a patient's teeth, wherein the patient's teeth are in an arrangement at a previous time corresponding to an end of a previous orthodontic treatment;
registering the patient's teeth in the first three-dimensional virtual model with the patient's teeth in the second three-dimensional virtual model;
generating an orthodontic virtual setup for a subsequent orthodontic treatment of the patient's teeth.

2. The method of Claim 1, wherein the step of registering comprises matching features of the patient's teeth in the first three-dimensional virtual model with features of the patient's teeth in the second three-dimensional virtual model.

3. The method of Claim 1 or 2, wherein the step of registering comprises using intercuspal positions of the patient's teeth in the first and second three-dimensional virtual models.

4. The method of any of Claims 1 to 3, wherein the step of registering comprises an iterative method to remove part of the teeth that are not in the same position in the first and second three-dimensional virtual models.

5. The method of any of Claims 1 to 4, wherein the step of registering comprises the steps of globally registering the first three-dimensional virtual model of the patient's teeth with the second three-dimensional virtual model of the patient's teeth and registering the first and second three-dimensional virtual models of the patient's teeth on a tooth-by-tooth basis.

6. The method of any of Claims 1 to 5, wherein the method further comprises a step of generating intermediate orthodontic treatment steps corresponding to a series of aligners for moving the patient's teeth into an arrangement of the orthodontic virtual setup.

7. The method of any of Claims 1 to 6, wherein the step of generating an orthodontic virtual setup comprises determining whether collisions exist for each registered tooth with each of the adjacent teeth.

8. The method of Claim 7, wherein the step of determining comprises computing the amount of movement necessary for each registered tooth to move from its position in the first three-dimensional virtual model to its position in the second three-dimensional virtual model.

9. The method of Claim 8, wherein the step of determining further comprises comparing the amount of movement with a pre-determined, pre-decided threshold value.

10. The method of any of Claims 7 to 9, wherein the method further comprises a step of moving any teeth involved in a collision slightly to avoid the collision.

11. The method of any of Claims 1 to 10, wherein the step of obtaining a first three-dimensional virtual model of the patient's teeth comprises collecting three-dimensional scan data of the patient's teeth through use of a cone beam computed tomography device.

12. The method of any of Claims 1 to 11, wherein the step of obtaining a first three-dimensional virtual model of the patient's teeth comprises collecting three-dimensional scan data of the patient's teeth through use of an intraoral scanner.

13. The method of any of Claims 1 to 12, wherein the step of obtaining a second three-dimensional virtual model of the patient's teeth comprises retrieving a three-dimensional virtual model of the patient's teeth created at the end of a previous orthodontic treatment of the patient's teeth.
